# EUROPEAN PATENT SPECIFICATION

(11) **EP 3 666 249 B1**
(45) Date of publication and mention of the grant of the patent: **08.09.2021**
(21) Application number: 18460073.2
(22) Date of filing: 17.12.2018
(51) Int. Cl.: A61K 8/58, A61Q 19/06, A61K 31/133, A61K 31/69, A61K 31/695, C07F 7/08, A61K 8/41, A61Q 19/08

(54) **COMPLEXES OF ORGANIC SILICON COMPOUNDS WITH BORON CITRATE AND DIMETHYLETHANOLAMINE**
KOMPLEXE VON ORGANISCHEN SILIKONVERBINDUNGEN MIT BORONCITRAT UND DIMETHYLETHANOLAMIN
COMPLEXES DE COMPOSES DE SILICIUM ORGANIQUES AVEC DU CITRATE DE BORE ET DE DIMETHYLETHANOLAMINE

(30) Priority: 10.12.2018 PL 42811818
(43) Date of publication of application: 17.06.2020
(73) Proprietor: Jednostka Innowacyjno-Wdrozeniowa INWEX Spolka z Ograniczona odpowiedzialnoscia, 25-323 Kielce (PL)
(72) Inventor: SZCZEPANIAK, Stanislaw, Kielce (PL); SZCZEPANIAK, Remigiusz, Kielce (PL); SZCZEPANIAK, Monika, Kielce (PL); SZCZEPANIAK, Elwira, Kielce (PL); SZCZEPANIAK, Dominika, Kielce (PL)
(74) Representative: Basa, Grazyna

(56) References cited:
- S SZCZEPANIAK ET AL: "TI -New organosilicon boron citrate complexes and process for the preparation thereof", PL214750 B1, 1 January 2013 (2013-01-01), pages 1-4, XP055565133,

## Description

The subject matter of the invention are complex organic silicon compounds, method of obtaining complex organic silicon compounds and use of complex organic silicon compounds.

Specialist and patent literature mentions citrate organic silicon complexes. In order to prevent olimerization in aqueous solutions of organic silicon compounds, they are stabilised with citric acid or its salts, and then used as the most easily absorbed dietary supplements and for therapeutic and cosmetic purposes.

For example, patent specification US 3 914 416 describes obtaining methylsilanetriol complexes with salicylic acid or mannuronic acid in aqueous solutions from pH 1 to 8. The above mentioned stabilised silicon complexes are applied in various cosmetics and as therapeutic preparations.

Another patent specification US 4 994 445 presents aqueous compositions for treatment of the cardiovascular system and demonstrating good therapeutic action. Aqueous composition consists of organic silicon stabilized with citric acid, salicylic acid or other organic acids. Additionally, the therapeutic composition contains at least one metal selected from the group including titanium, zirconium, vanadium, chrome, rhodium, gold, iridium, platinum group metals and others.

Patent specification US 5 391 546 describes aqueous therapeutic compositions comprised of organic silicon compounds stabilized with citric acid or salicylic acid and sodium thiosulphate or magnesium thiosulphate.

Patent specification FR 2 684 002 presents aqueous compositions containing organic silicon stabilized with one or more organic acids or their salts to pH ranging from 3.5 to 8, used for treating virus diseases. Examples of execution indicate that citric acid is used to stabilize organic silicon. The above composition for treatment of virus diseases involves organic and inorganic sulphur compounds excluding thiosulphate.

Patent specifications GB 955 969 and GB 1 206 790 describe methods of obtaining organic silicon complexes. In order to obtain organic silicon complexes salicylic acid and its derivatives, citric acid or their salts are used. PH of the above complexes is determined by treatment with ion exchange resins.

Polish patent PL 214750 presents complex organic silicon compounds with boron citrate, which for proper pH level, preferably 5.5 - 8.0, are corrected with acids or alkalis which are harmless for humans and animals. Those alkalis are monovalent metals.

For years inventors have searched for new, bio-absorbed complexes with organic silicon, which would be stable in a broad range of pH levels, non-toxic for living organisms and at the same time having biocidal, preservative and regenerating effect on the human skin.

The above searched compound which forms stable complex compounds with organic silicon is boron citrate with molecular formula C₁₈H₁₅BO₂₁ and molecular weight 578.1, which a well-known chemical compound, manufactured by many companies for various industrial applications. Boron citrate 5% - a powder which contains 5% of boron per boron content, it is non-toxic for humans; only doses above 500mg/human/day may cause problems of the digestive system such as: nausea, diarrhoea, loss of appetite, and others.

The state of the art includes also dimethylethanolamine which is an organic chemical compound from the amino-alcohol group. In human body dimethylethanolamine is a precursor for acetylcholine synthesis and is used as a drug stimulating the central nervous system - it enhances the ability to focus as well as psychological and physical fitness, decreases the demand for sleep, calms down and improves the mood. Additionally, dimethylethanolamine has anti-inflammatory and firming effect on the skin. Organic and inorganic salts of dimethylethanolamine are used for production of dietary supplements and in cosmetics.

On 9 March 2016 the European Food Safety Authority (EFSA) permitted only monomethylsilanetriol in concentrations of 100-150 mg Si/l as the new food ingredient from the entire group of organic silicon compounds.

Therefore, the below examples are limited only to monomethylsilanetriol in low concentrations of organic silicon per litre.

Unexpectedly, it turned out that the compound in the form of complex organic silicon compounds with boron citrate and dimethylethanolamine is especially useful for treatment of various diseases and in cosmetics, in particular for regeneration of the epidermis. The problem of epidermal regeneration is particularly important in case of cosmetic treatments with the use of laser technology which are becoming more and more popular and which are performed at many beauty salons as a surface treatment correcting wrinkles, cellulite, stretch marks and scars.

The subject of invention are complex organic silicon compounds with boron citrate and dimethylethanolamine are presented by means of the following general formula:

[CH₃Si(OMe)₃]ₘ • [C₁₈H₁₅BO₂₁]n • [DMAE]ₚ

wherein Me is an alkaline, monovalent metal or hydrogen, DMAE is dimethylethanolamine, m has the value of 1-3. n has the value of 1-20, p has the value of 10-100.

The method of obtaining complex organic silicon compounds is characterised by the fact that alkaline monomethylsilanetriol is added in a continuous manner or in portions to aqueous solution of boron citrate in the molar ratio of 3-1 do 1-20, at the temperature from 0 to 90°C, and then the obtained aqueous solution of organic silicon and boron compounds with neutralised by means of dimethylethanolamine to a relevant pH level, preferably 5.0 - 9.0.

The invention consists a complex organic silicon compounds as defined above in the form of an independent drug or ingredient of pharmaceutical products, for use in dietary supplements and in the form of an independent cosmetic or in the form of an ingredient in cosmetic products.

According to the invention, the new obtained citrate-boron-dimethylethanolamine organic silicon complexes are non-toxic and additionally they contain organic boron and dimethylethanolamine necessary for living organisms. They may be applied as valuable silicon and boron supplements for humans, animals and birds.
According to the invention, the new obtained citrate-boron-dimethylethanolamine organic silicon complexes will be applied in treatment of various skin diseases, wounds, bedsores, and in fighting various bacterial, fungal and viral infections.
According to the invention, the new obtained citrate-boron-dimethylethanolamine organic silicon complexes will be applied in cosmetics as toners, ointment, shampoo, hair and nail conditioners as well as other skin, nail and hair care products.

It is beneficial to add to the compounds obtained in such manner the microelements necessary for proper development of humans, animals and birds, such as magnesium, calcium, potassium, sulphur, iron, chrome, zinc, copper, manganese, cobalt, molybdenum, selenium, silver, titanium, vanadium, gold, light and heavy platinum group metals, and others.

It is also beneficial to add compounds complexing calcium, magnesium and other metals contained in water. The compounds complexing the above metals include aminocarboxylic, aminoaspartic, aminohydroxyaspartic, aminophosphoric, phosphoniccarboxylic, polycarboxylic, alkil-hydroxycarboxylic, hydroksycarboxylic and other salts and/or acids.

According to the invention, it is beneficial to add to the new obtained citrate-boron-dimethylethanolamine organic silicon complexes natural and/or synthetic macromolecular compounds such as polysilicates, polyphosphates, carpenter's glue, gelatine, peptone, various plant and animal protein hydrolizates, starch, cellulose and its derivatives, polyvinyl alcohol, polyvinylpyrrolidone, copolymers and homopolymer of acrylic acid and/or metaacrylic acid or other unsaturated compounds, polyasparagines, polyglutamates, and others.

According to the invention, it is beneficial to add to the new obtained citrate-boron-dimethylethanolamine organic silicon complexes anionic, non-ionic, cationic and/or amphoteric surfactants. In order to reduce surface tension of aqueous solutions, disperse the compounds of silicon, ingredients of ointments, creams and shampoos which are poorly soluble in water as well as other ingredients which are non-soluble or poorly soluble in water and, what is the most important, for better penetration inside the human skin.

According to the invention, it is beneficial to add to the new obtained citrate-boron-dimethylethanolamine organic silicon complexes organic solvents mixing with water in a quantity up to 40% by weight, preferably lower aliphatic alcohols such as ethyl alcohol, propyl alcohol, isopropyl alcohol, dimethyl sulfoxide, aliphatic glycols, polyethylene glycols and/or propylene products of ethoxylation of glycerin and sugars as well as other solvents which are non-toxic for humans and animals.

According to the invention, it is beneficial to add to the new obtained citrate-boron-dimethylethanolamine organic silicon complexes organic the known biocidal compounds, beneficial sulphonamides, antibiotics, dithiocarbamates, izotiazolone, phenol and its derivatives, thiopyridines and its derivatives, benzoic acid and its derivatives.

According to the invention, it is beneficial to add to the new obtained citrate-boron-dimethylethanolamine organic silicon complexes inorganic sulphur compounds such as thiosulphates, sulphites, pyroulphites.

According to the invention, it is beneficial to add to the new obtained citrate-boron-dimethylethanolamine organic silicon complexes oxidizing compounds such as: H₂O₂, calcium and magnesium peroxides, persulphates, chlorates, perchlorates, chlorites, bromates, iodates, iodine, and others.

According to the invention, it is beneficial to add to the new obtained citrate-boron-dimethylethanolamine organic silicon complexes taste and flavour substances in quantities up to 1% by weight.

According to the invention, it is beneficial to add to the new obtained citrate-boron-dimethylethanolamine organic silicon complexes organic colourants in quantities up to 0.1% by weight.

The subject matter of the invention will be explained in detail in the below examples without limiting its scope.

### Example 1

To a glass reactor equipped with a high-speed stirrer 800g of distilled water was added at room temperature and then 5.0g of boron citrate. After the ingredients were solved, 25% solution of potassium monomethylsilanetriol was dosed in portions in the quantity of 3g. After the ingredients were mixed, pH was adjusted with dimethylethanolamine to be 8.5. Quantity
of the dimethylethanolamine used was 10.5g. The entire mixture was supplemented with distilled water up to the volume of 1 dm³. The organic boron content in the obtained solution amounts to 0.25 g, which corresponds to 23.13 mM, and the organic silicon content amounts to 0.10g, which corresponds to 3.6mM. 14 g dimethylethanolamine corresponds to 118 mM. The molar ratio of silicon to boron to dimethylethanolamine is 1: 6.4 : 32.8.

The above sample was frozen and thawed multiple times. After each thawing the sample was clear without any opacity or precipitation, which is the evidence of high stability of organic silicon in accordance with the invention.

### Example 2

As in example 1, to a glass reactor 850g of technological water was added at the temperature of 85°C and hardness of 18°N, then 2g of ethylenediaminetetraacetic acid disodium salt (Na₂EDTA) and 3.5g of boron citrate was added. After mixing the ingredients, while stirring intensively, 2.5g of 25% solution of potassium monomethylsilanetriol was added. After mixing the ingredients, pH was adjusted to be 6.5 by means of 10% dimethylethanolamine, of which 45 g were used. The entire mixture was supplemented with technological water up to the volume of 1dm³. The silicon content in the obtained sample amounts to 100.8 mg, which corresponds to 3.6 mM, while the boron content amounts to 175mg, which corresponds to 16.2 mM, and dimethylethanolamine content to 4.5 g, which corresponds to 50.8mM. From the above it follows that the molar ratio of silicon to boron and dimethylethanolamine is 1: 4.5 : 14. The above sample was frozen and thawed multiple times. After each thawing the sample was colourless and clear without any opacity or precipitation, which is the evidence of high stability of organic silicon in accordance with the invention.

### Example 3

As in example 1, to a reactor 790g of distilled water was added at the temperature of 35°C and 5.5g of boron citrate was added. Then, awhile stirring intensively, 25% solution of potassium monomethylsilanetriol was dosed in the form of drops in the quantity of 3.4 g. After mixing the ingredients, pH of reagents was adjusted to be 6.8 by means of 10% dimethylethanolamine, of which 65 g were used. The entire mixture was supplemented with distilled water up to the volume of 1dm³. The organic boron content in the obtained solution amounts to 275 mg, which corresponds to 25.46 mM, and the organic silicon content amounts to 120 mg, which corresponds to 4.1 mM; 6.5 g of dimethylethanolamine content corresponds to 73 mM.

From the above it follows that the molar ratio of organic silicon to boron to dimethylethanolamine is 1: 6.2: 17.8.

The above citrate-boron-dimethylethanolamine organic silicon complexes were frozen and thawed multiple times. After each thawing the sample was clear, without any opacity or precipitation, which is the evidence of high stability of organic silicon complexes.

### Example 4

As in example 1, to a reactor 700 g of demineralised water was added at the temperature of 65°C, then 1.2 g of ethylenediaminetetraacetic acid disodium salt (Na₂EDTA) and 5.0 g of boron citrate was added. While stirring intensively, 20% solution of sodium monomethylsilanetriol was dosed in the quantity of 4.2 g. After mixing the ingredients, pH was adjusted to be 8.5 by means of 15% dimethylethanolamine, of which 112 g were used. The entire mixture was supplemented with demineralized water up to the volume of 1 dm³. The organic boron content in the obtained solution amounts to 250 mg, which corresponds to 23.15 mM, and the organic silicon content in the obtained solution amounts to 110 mg, which corresponds to 3.9 mM; 16.8 g of dimethylethanolamine content corresponds to 188.7 mM. From the above it follows that the molar ratio of organic silicon to boron to dimethylethanolamine is 1: 1: 5.9: 48.4.

The above citrate-boron-dimethylethanolamine organic silicon complexes were frozen and thawed multiple times and each time the sample was clear, without any opacity, which is the evidence of high stability of organic silicon in accordance with the invention.

### Example 5

As in example 1, to a reactor 750g of demineralized water was added at the temperature of 5°C and 1.2g of boron citrate was added. After the ingredients were solved, while stirring intensively, 25% solution of potassium monomethylsilanetriol was dosed in portions in the quantity of 2.4g. After mixing the ingredients, pH was adjusted to be 5.5 by means of dimethylethanolamine, of which 4 g were used. Next, while stirring continuously, 2.0 g of 10% silver thiosulphate complex solution with the following formula: [Ag(S₂O₃)₂]⁻³ was added. The entire mixture was supplemented with distilled water up to the volume of 1dm³.

Concentrations of individual elements in the above synthesized sample are as follows: organic boron - 0.06g, which corresponds to 5.5mM, silicon content - 0.08, which corresponds to 2.85mM, and dimethylethanolamine content - 4 g, which corresponds to 44.9mM. The molar ratio of silicon to boron to dimethylethanolamine is 1: 1.9 : 15.7. The above sample was frozen and thawed multiple times and each time the sample was clear without any precipitation, which is the evidence of high stability of organic silicon in accordance with the invention.

### Example 6

As in example 1, to a reactor 700 g of distilled water at the temperature of 75°C was added, then 2.2 of boron citrate and 0.2 of magnesium carbonate (analytical grade chemical).

After the ingredients were solved, while stirring intensively, 3.1g of 45% sodium monomethylsilanetriol solution was added slowly. After mixing the ingredients, pH was adjusted to be 7.6 by means of dimethylethanolamine, of which 88g were used. The entire mixture was supplemented with distilled water up to the volume of 1 dm³. The organic boron content in the obtained solution amounts to 0.11g, which corresponds to 10.2 mM, organic silicon content amounts to 0.24g, which corresponds to 8.7 mM, while dimethylethanolamine content amounts to 8.8g, which corresponds to 98.8mM. The molar ratio of silicon to boron to dimethylethanolamine is 1: 1.2: 11.6.

The above sample was frozen and thawed multiple times and each time the sample was clear without any precipitation, which is the evidence of high stability of organic silicon in accordance with the invention.

### Example 7

As in example 1, to a reactor 900g of demineralized water was added at the temperature of 45°C and 4.4g of boron citrate was added. After the ingredients were solved, while stirring intesively, 22% solution of sodium monomethylsilanetriol was dosed in the quantity of 3.8g. After mixing the ingredients, pH was adjusted to be 8.8 by means of 20% dimethylethanolamine, of which 98g were used. The entire mixture was supplemented with water up to the volume of 1dm³. The boron content in the obtained preparation amounts to 220mg, which corresponds to 20.4mM, while the silicon content amounts to 110 mg, which corresponds to 3.9 mM, while dimethylethanolamine content amounts to 19.6g, which corresponds to 220.8mM. From the above it follows that the molar ratio of silicon to boron and dimethylethanolamine is 1: 5.2: 55.5.

The above sample was frozen and thawed multiple times and each time after thawing the sample was clear, without any opacity or precipitation, which is the evidence of high stability of organic silicon in accordance with the invention.

### Example 8

Application in cosmetics for reduction of wrinkles on the skin.

As in example 1, the citrate-boron-dimethylethanolamine organic silicon complex was mixed with arginine. The obtained solution in the form of a mist was applied by means of oxygen infusion to the skin area subject to testing.

The participants of the test were 22 persons aged 30-60. For comparison purposes the participants were divided into two groups of 11 persons.

The first subgroup (11 persons) was subject to the action of the solution of citrate-boron-dimethylethanolamine organic silicon complexes with arginine in the form of a mist which were applied by means of oxygen infusion to the skin area subject to testing 1 a week. Skin analysis showed that 9 persons had a complexion with elastotic and post-sun wrinkles (40 y.o.), 1 person had a complexion with expressive, mimic wrinkles (about 30 y.o.), 1 person had a complexion with atrophic wrinkles (about 50 y.o.).

The second subgroup (11 persons) was subject to the action of the solution of citrate-boron-dimethylethanolamine organic silicon complexes with arginine in the form of a mist which were applied by means of oxygen infusion to the skin area subject to testing for the first three weeks once a week. Next, in the fourth week laser treatment was applied, after which a gel with citrate-boron-dimethylethanolamine organic silicon complexes was used, which was later given to the patients for the purpose of home care. The analysis showed that 7 persons had a complexion with elastotic and post-sun wrinkles (about 40 y.o), 2 persons had a complexion with expressive and mimic wrinkles (about 30 y.o.), 2 persons had a complexion with atrophic wrinkles (about 50 y.o.).

In the scope of all treatments, the treatment parameters were selected individually for each patient, in accordance with the table below:

| | ***SKIN PHOTOTYPE I-III*** | | | ***SKIN PHOTOTYPE IV*** | | |
|---|---|---|---|---|---|---|
| ***TREATED AREA*** | energy | density | transitions | energy | density | transitions |
| ***cheeks*/ *forehead*** | 80 mJ - 160 mJ | 2-3 | 2-3 | 60 mJ - 120 mJ | 1-2 | 1-2 |
| ***eye rea* / *nose; deep changes*** | 120 mJ - 160 mJ | 2-3 | 2-3 | 80 mJ - 120 mJ | 1-2 | 1-2 |
| ***upperllower eyelid, lip area*** | 60 mJ - 160 mJ | 1-3 | 2 | 60 mJ - 120 mJ | 1-2 | 1-2 |
| | 80 mJ - 160 mJ | 1-3 | 2 | 60 mJ - 120 mJ | | |

The series of treatments with the use of citrate-boron-dimethylethanolamine organic silicon complexes with arginine, which were applied by means of oxygen infusion performed once a week for the period for three weeks, had a significant impact on moisturisation of the epidermis. Proper moisturisation makes the skin more elastic and firm after completion of the anti-wrinkle therapy. The treatment had a lesser effect on elimination or reduction of wrinkles - its effectiveness is assessed at the level of 36.4%. The series of treatments with the use of citrate-boron-dimethylethanolamine organic silicon complexes with arginine, which were applied by means of oxygen infusion performed in combination with fraction laser treatment had a significant effect on increasing the moisturisation and elasticity of skin. Additionally, the combination of oxygen infusion with fraction laser turned out to be highly effective in elimination of wrinkles. The effectiveness of the treatment in the scope of elimination of wrinkles was about 73% and its effects were comparable to at least two fraction laser treatments.

### Example 9

Application in reduction of lipodystrophy (cellulite).

As in example 1, the citrate-boron-dimethylethanolamine organic silicon complexes were mixed with caffeine. The obtained mixture in the form of a gel was applied by means of ultrasounds (cavitation liposuction).

The participants of the test were 22 persons aged 30-60. All participants of the test were divided into two groups of 11 persons. The first subgroup was subject to three treatments with the use of citrate-boron-dimethylethanolamine organic silicon complexes mixed with caffeine in the form of a gel applied by means of ultrasounds (cavitation liposuction) 1 a week. The analysis showed that 4 persons had 2nd degree cellulite, 4 persons had 1st degree cellulite, and 3 persons had 3rd degree cellulitis (according to the *Nurnberger-Müller* scale). The second subgroup was subject to the action of citrate-boron-dimethylethanolamine organic silicon complexes mixed with caffeine in the form of a gel applied by means of ultrasounds (cavitation liposuction); the treatments were performed once a week for the first three weeks. In the fourth week Nd;Yag 1064 nm "Genesis" laser was applied. After the treatments a gel with citrate-boron-dimethylethanolamine organic silicon complexes was used in the form of a gel, which was later given to the patients for the purpose of home care. The analysis showed that 6 persons had 2nd degree cellulite, and 5 persons had 3rd degree cellulitis (according to the *Nurnberger-Müller* scale).

For the purpose of comparison of the effects of therapy the following was taken into account: degree of intensity of the effect (according to the Nürnberger-Müller scale), measurement of the size of the tested part of the body, high-frequency ultrasound examination. The result of effectiveness of the anti-cellulite therapy is demonstrated by smoothing out the boundary between the subcutaneous tissue and dermis as well as reducing the thickness of the subcutaneous tissue. Decrease in the value of those two parameters proves the effectiveness of the anti-cellulite therapy and improvement of the skin condition.

The series of treatments with the use of ultrasound method of application of the active substance of citrate-boron-dimethylethanolamine organic silicon complexes in the form of a gel with caffeine on visual inspection and on palpation had a positive impact on moisturisation of the epidermis and increase of elasticity of the skin in the tested group. The effectiveness of the method in elimination of the degrees of intensity of the defect (on the basis of an ultrasound examination) was at the level of 54.5% in the tested group. This means that the therapy was effective for more than half of the tested persons (in the subgroup). The treatment series with the use of active substance with laser (Genesis Nd:Yag 1064 nm laser head) on visual inspection and on palpation had a significant effect on moisturisation and elasticity of the epidermis. The effectiveness of the method in elimination of the degree of reduction of the defect (on the basis of an ultrasound examination) was at the level of 72.7% in the tested subgroup.

## Claims

1. A complex organic silicon compounds with boron citrate and dimethylethanolamine of formula:
[CH₃Si(OMe)₃]ₘ • [C₁₈H₁₅BO₂₁]ₙ • [DMAE]ₚ
wherein:
Me is an alkaline, monovalent metal or hydrogen;
DMAE is dimethylethanolamine;
m has the value of 1-3;
n has the value of 1-20;
p has the value of 10-100.

2. A method of obtaining complex organic silicon compounds of claim 1 is **characterised by** the fact that alkaline monomethylsilanetriol is added in a continuous manner or in portions to aqueous solution of boron citrate in the molar ratio of 3-1 do 1-20, at the temperature from 0 to 90°C, and then the obtained aqueous solution of organic silicon and boron compounds with neutralised by means of dimethylethanolamine to a relevant pH level, preferably 5.0 - 9.0.

3. A complex organic silicon compounds according to claim 1 in the form of an independent drug or ingredient of pharmaceutical products, for use in dietary supplements and in the form of an independent cosmetic or in the form of an ingredient in cosmetic products.

## Patentansprüche

1. Komplexe organische Verbindungen von Silicium mit Borcitrat und *N,N-*Dimethylethanolamin mit der Formel:
[CH₃Si(OMe)₃]ₘ • [C₁₈H₁₅BO₂₁]n • [DMAE]ₚ
wo:
Me - ein alkalisches einwertiges Metall oder Wasserstoff darstellt;
DMAE - Dimethylethanolamin darstellt;
m den Wert von 1 bis 3 aufweist;
n den Wert von 1 bis 20 aufweist;
p den Wert von 10 bis 100 aufweist.

2. Methode der Gewinnung von komplexen organischen Siliciumverbindungen nach dem Anspruch 1, **dadurch gekennzeichnet, dass** zur wässrigen Lösung vom Borcitrat alkalisches Monomethylsilanetriol dauernd oder portionsweise zugegeben wird, um das Molverhältnis von 3-1:1-20 in der Temperatur von 0 bis 90 °C zu erhalten. Woraus wir eine wässrige Lösung von organischen Silicium- und Borverbindungen erhalten, die mittels Dimethylethanolamin bis zum entsprechenden pH-Wert, vorzugsweise 5,0 - 9,0, neutralisiert wird.

3. Komplexe organische Siliciumverbindungen nach dem Anspruch 1 in Form eines separaten Arzneimittels oder Inhaltsstoffes in Pharmaprodukten, zur Nutzung in **Nahrungsergänzungsmitteln** und in Form eines separaten Pflegemittels oder Inhaltsstoffes in Pflegemittelprodukten.

## Revendications

1. Composés organiques complexes de silicium avec du citrate de bore et de la diméthyléthanolamine à formule :
[CH₃Si(Ome)3]m₃]m [C₁₈H₁₅BO₂₁]n [DMAE]ₚ
où:
le Me est un métal alcalin, un métal monovalent ou l'hydrogène ;
le DMAE est une diméthyléthanolamine :
le m a une valeur se situant entre 1 et 3 ;
le n une valeur se situant entre 1 et 203 ;
le p a une valeur se situant entre 10 et 100.

2. Procédé de préparation de composés organiques complexes de silicium selon la revendication 1, **caractérisé en ce que** le monométhylsilanetriol alcalin est ajouté en continu ou par portions à la solution aqueuse de citrate de bore pour obtenir un rapport molaire de 3-1:1-20, à une température de 0 à 90 °C, puis la solution aqueuse obtenue des composés organiques du silicium et du bore est neutralisée avec de la diméthyléthanolamine à un pH approprié, de préférence 5,0-9,0.

3. Composés organiques composites de silicium selon la revendication 1 sous la forme d'un médicament unique ou d'un composant des produits pharmaceutiques, pour être utilisé dans des compléments alimentaires et comme composant d'un produit de beauté.
